# EUROPEAN PATENT SPECIFICATION

(11) **EP 4 114 962 B1**
(45) Date of publication and mention of the grant of the patent: **22.01.2025**
(21) Application number: 21709698.1
(22) Date of filing: 08.03.2021
(51) Int. Cl.: C12Q 1/04, C12Q 1/18, C12Q 1/34, C12Q 1/66, G01N 33/542

(54) **SOLID COMPOSITION COMPRISING LUCIFERASE, D-LUCIFERIN, NUTRIENTS AND AGAR.**
FESTE ZUSAMMENSETZUNG BESTEHEND AUS LUCIFERASE, D-LUCIFERIN, NÄHRSTOFFEN UND AGAR.
COMPOSITION SOLIDE COMPRENANT DE LA LUCIFÉRASE, DE LA D-LUCIFÉRINE, DES NUTRIMENTS ET DE LA GÉLOSE.

(30) Priority: 06.03.2020 EP 20161528
(43) Date of publication of application: 11.01.2023
(73) Proprietor: Biosynth AG, 9422 Staad (CH)
(72) Inventor: IHSSEN, Julian, 9014 St. Gallen (CH); SPITZ, Urs, 9010 St. Gallen (CH)
(74) Representative: Fritsche, Erich Roland
(86) International application number: PCT/EP2021/055793
(87) International publication number: WO 2021/176101

(56) References cited:
- WO-A1-97/19353
- SE-B- 374 433
- US-B1- 7 132 249
- "CURRENT TOPICS IN MEMBRANES", vol. 54, 1 January 2003, SAN DIEGO,CA, US, ISSN: 1063-5823, article ERIK M SCHWIEBERT ET AL: "Cellular Mechanisms and Physiology of Nucleotide and Nucleoside Release from Cells: Current Knowledge, Novel Assays to Detect Purinergic Agonists, and Future Directions", pages: 31 - 58, XP055687735, DOI: 10.1016/S1063-5823(03)01002-0
- LENNART NILSSON: "New Rapid Bioassay of Gentamicin Based on Luciferase Assay of Extracellular ATP in Bacterial Cultures", ANTIMICROBIAL AGENTS AND CHEMOTHERAPY, vol. 14, no. 6, 1 January 1978 (1978-01-01), pages 812 - 816, XP055688285
- VALAT CHARLOTTE ET AL: "Use of ATP bioluminescence to determine the bacterial sensitivity threshold to a bacteriocin", LUMINESCENCE: THE JOURNAL OF BIOLOGICAL AND CHEMICAL LUMINESCENCE; [31660], JOHN WILEY & SONS LTD, GB, vol. 18, no. 5, 1 January 2003 (2003-01-01), pages 254 - 258, XP002336963, ISSN: 1522-7235, DOI: 10.1002/BIO.735
- LOMAKINA G YU ET AL: "Bioluminescence assay for cell viability", BIOCHEMISTRY, MAIK NAUKA - INTERPERIODICA, RU, vol. 80, no. 6, 18 June 2015 (2015-06-18), pages 701 - 713, XP035486768, ISSN: 0006-2979, [retrieved on 20150618], DOI: 10.1134/S0006297915060061
- TERRY RISS ET AL: "CHOOSING THE RIGHT CELL-BASED ASSAY FOR YOUR RESEARCH", CELL NOTES, ISSUE 6, 2003, PROMEGA, 1 January 2003 (2003-01-01), pages 6 - 12, XP055300610, Retrieved from the Internet <URL:https://www.fishersci.com/content/dam/fishersci/en_US/documents/programs/scientific/brochures-and-catalogs/publications/promega-choosing-cell-based-assay-publication.pdf> [retrieved on 20160907]
- SCHWIEBERT E M ET AL: "Extracellular ATP as a signaling molecule for epithelial cells", BBA - BIOMEMBRANES, ELSEVIER, AMSTERDAM, NL, vol. 1615, no. 1-2, 2 September 2003 (2003-09-02), pages 7 - 32, XP004451108, ISSN: 0005-2736, DOI: 10.1016/S0005-2736(03)00210-4
- NGUYEN DUNG T. ET AL: "The development of paper discs immobilized with luciferase/D-luciferin for the detection of ATP from airborne bacteria", SENSORS AND ACTUATORS B: CHEMICAL, vol. 260, 1 May 2018 (2018-05-01), NL, pages 274 - 281, XP055800621, ISSN: 0925-4005, DOI: 10.1016/j.snb.2018.01.009

## Description

### FIELD OF THE INVENTION

The present invention relates to a solid composition for preparing a solid agar growth medium by mixing the solid composition with an aqueous solution, the solid composition comprising luciferase, D-luciferin, nutrients, and agar. In addition, the present invention relates to the use of said solid composition in a method for the detection of ATP from cell cultures.

### BACKGROUND OF THE INVENTION

Luciferases are a class of oxidative enzymes, which are able to generate bioluminescent light by means of a chemical reaction with oxygen and a substrate. The most commonly used luciferases are the firefly luciferases. Firefly luciferase catalyzes a two-step oxidation reaction of its specific substrate, D-luciferin, in the presence of Mg²⁺, O₂ and ATP with characteristic light release kinetics. The first step is activation of luciferin by ATP to yield a reactive mixed anhydride (luciferyl-AMP). In the second step, the activated intermediate reacts with molecular oxygen to create a transient dioxetane (oxyluciferin) that breaks down to the oxidized products oxoluciferin and CO₂ under emission of visible light (530-640 nm) with a maximal intensity at 560 nm.

Luciferin-luciferase bioluminescence is a highly sensitive detection method used for numerous clinical, diagnostic and research applications. In almost every modern technique, bioluminescence has proven to be superior to traditional chromogenic, fluorogenic and radiolabeling methods. For example, radiolabeling in immunoassays has been almost entirely replaced by bioluminescence for obvious safety and practical reasons, while maintaining comparable levels of sensitivity. Fluorescence detection is also generally surpassed by bioluminescence in terms of sensitivity, selectivity and lower background noise.

The luciferin-luciferase system can, for example, be used in ATP assays, detection of enzyme activities, *in vitro* reporter gene assays, whole animal imaging (*in vivo* reporter assay), immunoassays, and pyrosequencing. However, the application of wild-type (WT) beetle luciferases are often limited by insufficient stability of these enzymes at temperatures above 30°C. In addition, the specific conditions of a particular assay may have an inhibiting effect on the luciferase reaction, thereby affecting the assays sensitivity. Therefore, many novel beetle luciferases with promising properties including increased thermostability have been developed in recent years.

A key application of the luciferin-luciferase system is the measurement of ATP in living (viable) cells. Adenosine triphosphate (ATP) is present in all living cells and plays a central role in the energy balance. The intracellular concentration of ATP is tightly regulated and is maintained at a similar level in all cells. When a cell dies, the ATP is completely degraded; ATP levels therefore reflect the presence of living cells. The ATP bioluminescence can therefore be used as a measure of growth (proliferation) of viable cells in culture and/or the impact of substances (e.g., cytotoxic agents or growth regulatory substances) on the viability of cells in culture. Under optimum conditions, light intensity is linearly related to ATP concentration. Cellular ATP is typically measured by lysis of the cells with suitable detergents, which leads to the release of ATP that in turn reacts with the luciferin-luciferase and results in detectable light emission.

Another important application of the luciferin-luciferase system is the detection of a broad range of enzymatic activities by using D-luciferin coupled with enzyme-labile groups as substrates (referred to as "pro-luciferins"). Pro-luciferins can be used, for example, for the detection of specific groups of bacteria expressing a target enzyme that is capable of cleaving the enzyme-labile group to release the luciferase substrate D-luciferin. Then, typically after lysis of the cells, in the presence of ATP, Mg²⁺ and oxygen present in an assay reaction mixture, the reaction of D-luciferin with luciferase leads to detectable light emission.

The cellular ATP-based cell viability assay and the enzyme activity assay using pro-luciferin substrates generally involve the following steps: 1) incubating a sample containing cells (e.g., bacterial cells) in a liquid culture medium, 2) cell lysis, 3) addition of lysate to luciferase/luciferin assay solution (luciferase, D-luciferin, Mg²⁺) or luciferase/pro-luciferin assay solution (luciferase, pro-luciferin, Mg²⁺, ATP), and 4) measurement of luminescence to quantify the number of viable cells (for ATP assay) or to detect the specific enzymatic activity of the target microorganism (for, e.g., the detection of specific groups of bacteria). In order to increase the ease of use and convenience of these assays, it would be desirable to simplify the steps and/or reduce the number of steps.

The assessment of cell viability also plays a key role in antibiotic susceptibility testing (AST). Antibiotic susceptibility testing is used to determine which antibiotic agent will impair cell viability, in particular cell growth. The results from this test will help a healthcare practitioner to determine which antibiotics are likely to be most effective in treating a person's infection. In addition, the results allows the practitioner to choose a narrow-spectrum antibiotic, thereby slowing down the development of antibiotic resistance caused by the incorrect use of broad spectrum antibiotics. Thus, it is increasingly important to specifically test for resistance to shorten treatment times, ensure efficacy and reduce the risk of antibiotic resistance.

The most frequently used AST methods are based on growth inhibition, wherein bacterial growth is assessed, either in agar, by measuring zones of inhibition around a disk or strip impregnated with antibiotic (e.g., by the Etest gradient diffusion method or the disk diffusion test) or by assessing turbidity in liquid media containing a drug (e.g., using the broth microdilution test). These methods, however, are time-consuming because bacteria from a patient's sample must initially be grown to reach a suitable concentration for testing, which typically takes about 18-24 hours. Once the bacteria reach a suitable concentration, the bacteria are exposed to different antibiotics and bacterial growth is determined after several hours of culturing (e.g., 12 hours), typically after overnight culturing. At the earliest, results can be available three days after the sample is taken. Thus, the biggest drawback of conventional growth dependent AST methods is that they are slow. In addition, the culturing can be difficult to ensure reproducibility. Furthermore, a given test can be limited in the number of antibiotics to be tested, in ease of use, costs and reliability.

US 7,132,249 B1 discloses a luciferin/luciferase tablet containing further substances such as lactose for the detection of ATP.

### OBJECT OF THE INVENTION

In view of the above, the object of the present invention is to provide improved methods for assessing cell viability.

### SUMMARY OF THE INVENTION

The above object is solved by the provision of a solid composition, comprising luciferase, D-luciferin, nutrients, and agar, for preparing a solid agar growth medium by mixing the solid composition with an aqueous solution. The solid composition can be used in a method for the detection of ATP from cell cultures, e.g., in a method for assessing cell viability or in a method for assessing susceptibility of bacterial cells to antibiotics.

In a first aspect, the present invention relates to a solid composition for preparing a solid agar growth medium by mixing the solid composition with an aqueous solution, the solid composition comprising luciferase, D-luciferin, nutrients (in particular one, two or all three of peptone, yeast extract and meat extract), and agar. The solid composition may optionally contain a buffer substance. The solid composition is, e.g., in the form of powders, beads, micronized particles, tablets, coatings, cuttable strips, pearls and the like.

Preferably, the solid composition comprises 0.01-0.10 wt.% luciferase, 0.02-0.40 wt.% D-luciferin, 30-70 wt.% agar, and one, two or all three of peptone, yeast extract and meat extract, and, optionally, a buffer substance.

Preferred embodiments of the present invention are set forth in the appended claims. Further embodiments and other objects, advantages and features of the present invention will become apparent from the following detailed description of the invention and the examples.

### BRIEF DESCRIPTION OF THE FIGURES

The present invention is further illustrated by reference to the following figures:

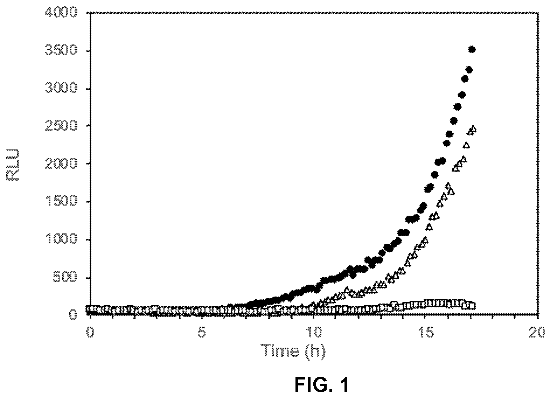

**FIG. 1** shows the results from an ATP assay for the detection of live bacterial cells on solid agar medium. Relative luminescence units (RLU) were measured over time for wells of a microplate containing a solid nutrient agar medium comprising luciferase and D-luciferin, which has been inoculated with samples of *Staphylococcus aureus* (open triangles), *Salmonella enteritidis* (closed circles), and sterile PBS (control, open squares). Initial cell number in the microplate wells was 10 CFU.

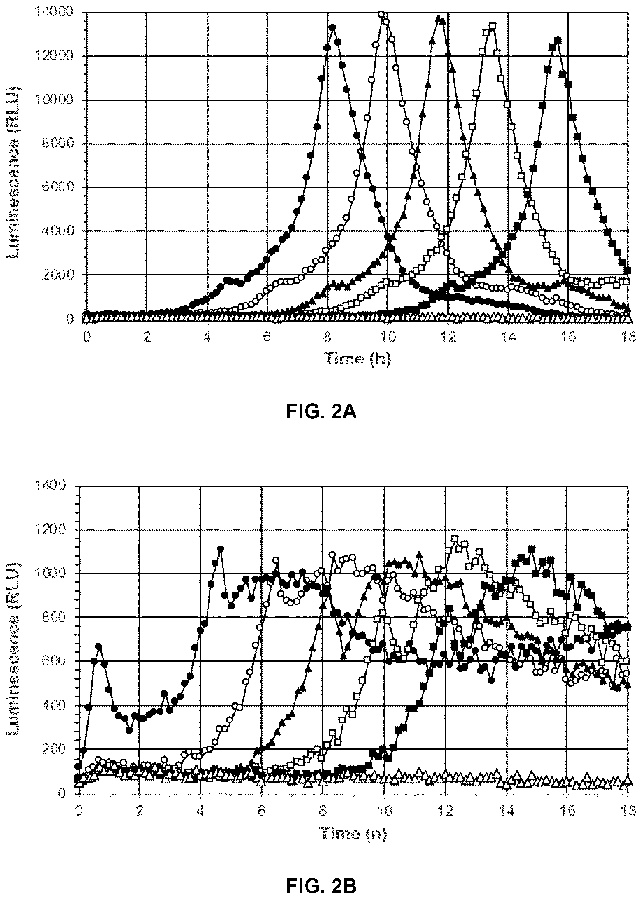

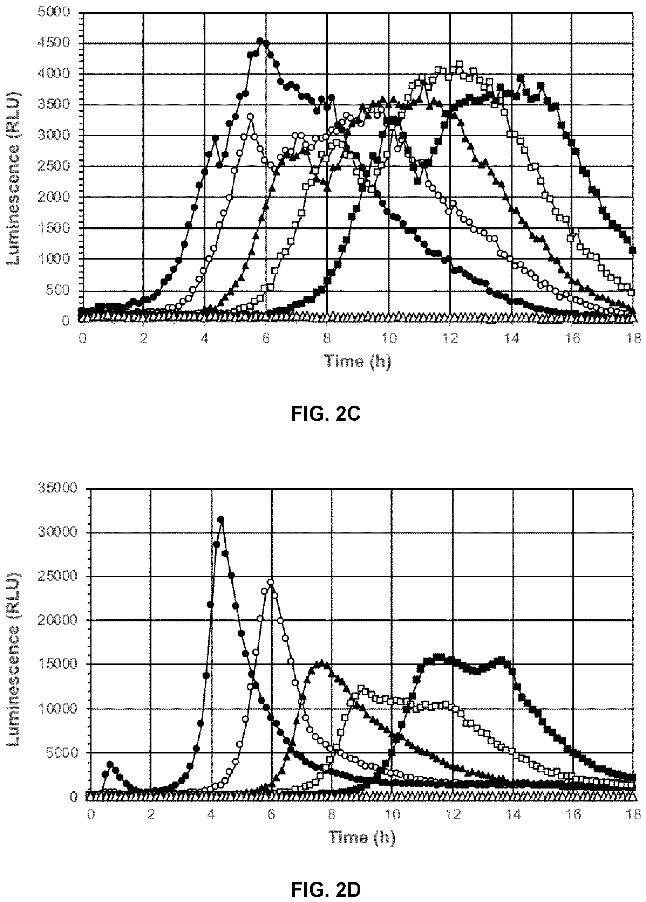

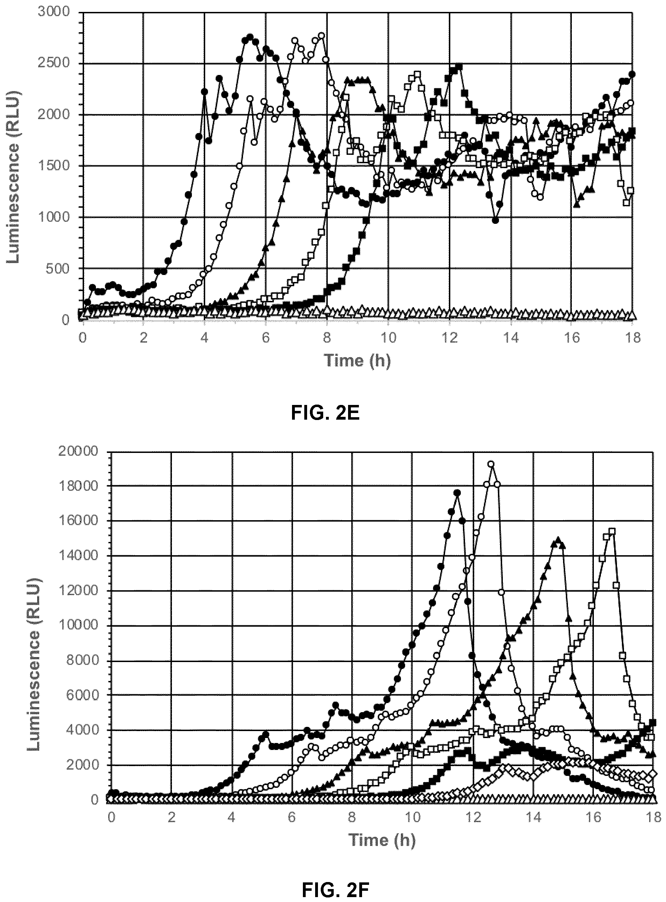

**FIG. 2** shows the results from an ATP assay for the detection of live bacterial cells in liquid medium. Relative luminescence units (RLU) were measured over time for wells of a microplate containing a liquid broth medium comprising luciferase and D-luciferin, which has been inoculated with serially diluted samples of five different bacterial strains and a mixture thereof. Initial cell concentrations in microplate wells were as follows: 10⁶ CFU/ml (closed circles), 10⁵ CFU/ml (open circles), 10⁴ CFU/ml (closed triangles), 10³ CFU/ml (open squares), 10² CFU/ml (closed squares), 0 (sterile control, open triangles). **FIG.2A****:** *Pseudomonas fluorescens,* **FIG. 2B**: *Pantoae agglomerans,* **FIG. 2C**: *Bacillus cereus,* **FIG. 2D**: *Staphylococcus aureus,* **FIG. 2E****:** *Escherichia coli,* **FIG. 2F****:** mixture of all five bacterial strains.

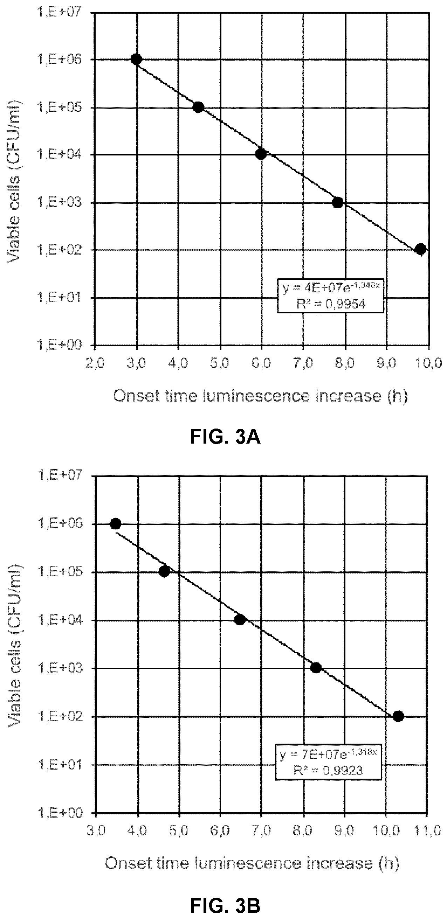

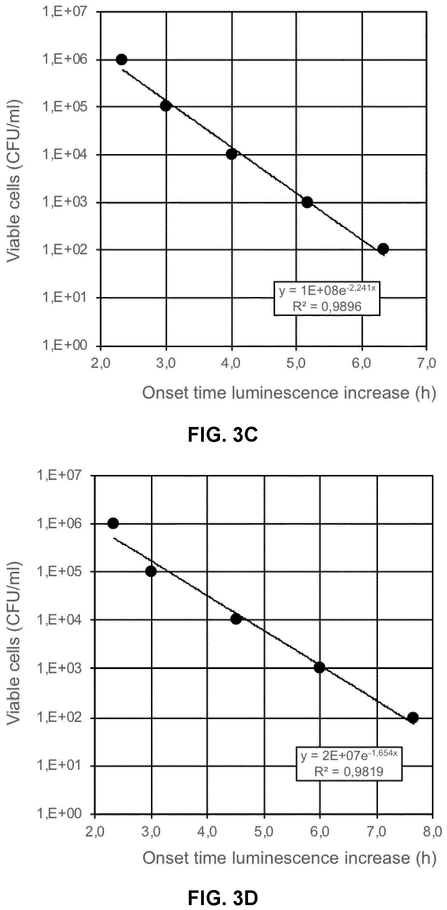

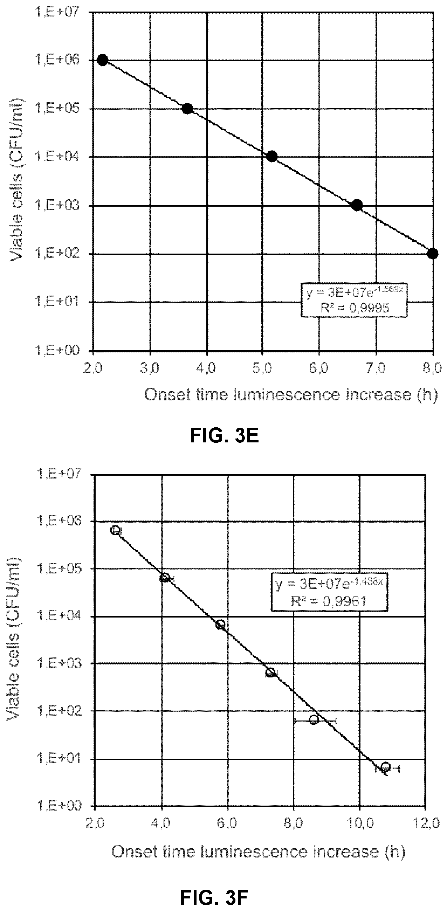

**FIG. 3** shows the correlation of onset times (start of exponential increase of relative luminescence units) with initial cell concentration for five different bacterial strains and a mixture thereof. **FIG. 3A**: *Pseudomonas fluorescence,* **FIG. 3B**: *Pantoae agglomerans,* **FIG. 3C**: *Bacillus cereus,* **FIG. 3D**: *Staphylococcus aureus,* **FIG. 3E****:** *Escherichia coli,* **FIG. 3F****:** mixture of all five bacterial strains (average values of three replicate experiments, error bars represent standard deviations).

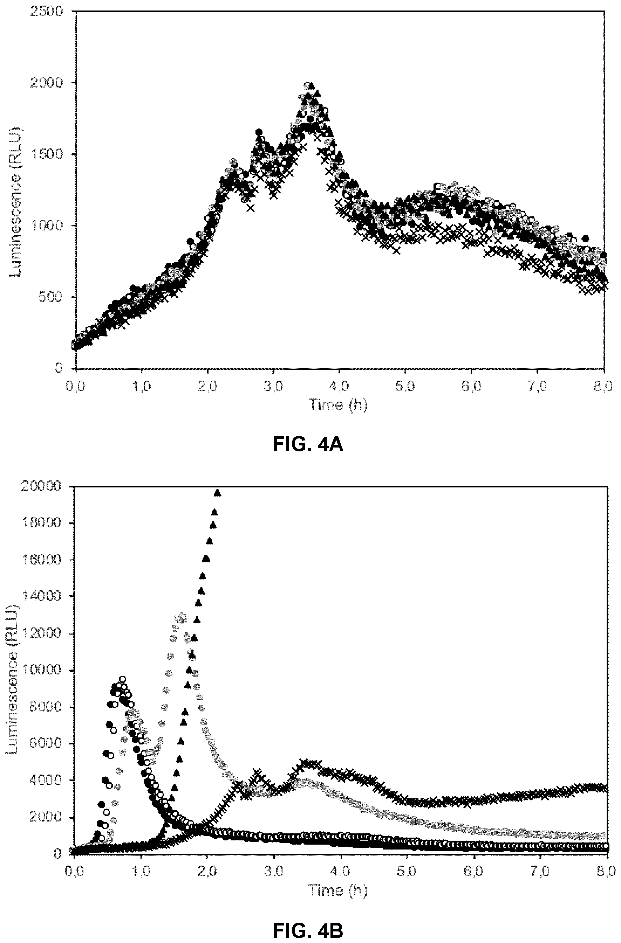

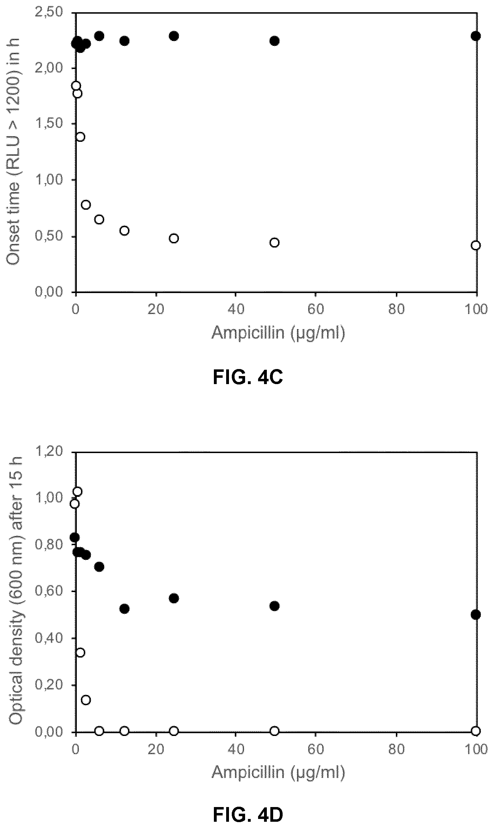

**FIG. 4** shows the results of an ATP assay for antibiotic susceptibility testing (AST) using ampicillin (AMP)-resistant (AMP^{R}) and AMP-susceptible (AMP^{S}) *Escherichia coli.* Relative luminescence units (RLU) were measured over time for wells of a microplate containing liquid broth medium comprising luciferase, D-luciferin and varying amounts of the antibiotic ampicillin, inoculated either with AMP^{R} or AMP^{S} E. *coli.* **FIG. 4A**: Amp^{R} *E*. *coli,* 100 µg/ml ampicillin (filled circles), 25 µg/ml ampicillin (open circles), 6.25 µg/ml ampicillin (shaded circles), 1.56 µg/ml ampicillin (filled triangles), and control without ampicillin (crosses); **FIG. 4B****:** Amp^{S} *E. coli,* same symbol designations as in Fig. 4A; **FIG. 4C**: onset time (first time point with RLU above 1200) in dependency of ampicillin concentration for Amp^{R} *E*. *coli* (closed circles) and Amp^{S} *E*. *coli* (open circles); **FIG. 4D**: optical density after 15 h in dependency of ampicillin concentration for Amp^{R} *E*. *coli* (closed circles) and Amp^{S} *E. coli* (open circles).

### DETAILED DESCRIPTION OF THE INVENTION

In a first aspect, the present invention relates to a solid composition for preparing a solid agar growth medium by mixing the solid composition with an aqueous solution, the solid composition comprising luciferase, D-luciferin, nutrients, agar, and optionally a buffer substance.

Preferably the solid composition comprises, or essentially consists of, 0.01-0.10 wt.%, preferably 0.02-0.06 wt.%, luciferase, 0.02-0.40 wt.%, preferably 0.04-0.20 wt.%, D-luciferin, 30-70 wt.%, preferably 40-60 wt.%, more preferably 45-55 wt.%, agar, nutrients, preferably one, two or all three of peptone, yeast extract and meat extract, and, optionally, a buffer substance. All wt.% relate to the total weight of the solid composition. The buffer substance is not limited in any way and may include phosphate buffer, MOPS 3-(N-morpholino)propanesulfonic acid) buffer and the like.

Within the present invention, the luciferase is generally used in the form of a lyophilized material, e.g., as lyophilized luciferase. The lyophilized luciferase material may consist of luciferase and, optionally, other substances such as stabilizing agents (e.g., human serum albumin, sucrose and the like).

As used herein, the term "nutrients" generally refers to substances or materials that are necessary for, or promote, the growth of cells, in particular of bacterial and/or fungal cells. In other words, the composition comprises nutrients which, when the composition is mixed with an aqueous solution such as water, provides a growth medium.

Furthermore, the term "nutrients" is intended to include "complex nutrients" and "minimal nutrients", i.e., the term "nutrients" may refer to "complex nutrients" or "minimal nutrients" or both. The term "complex nutrients" refers to raw materials containing two or more substances that are necessary for or that promote the growth of microorganisms. Examples of complex nutrients include natural raw materials such as peptone, yeast extract, meat extract and the like. Preferably, the composition according to the first aspect of the present invention contains one or two or all three of peptone, yeast extract and meat extract. A special advantage of these complex nutrients is the wide range of individual substances, such as amino acids, proteins, vitamins, mineral salts or trace elements, which can be made available to cultured cells, e.g., bacterial and/or fungal cells.

The term "minimum nutrients", as used herein, refers to chemically defined substances that include the minimum nutrients enabling the growth of the cultured cells, e.g., bacterial and/or fungal cells. Minimum nutrients typically contain a carbon source (e.g., glucose, pyruvate, succinate etc.), salts (e.g., MgSO₄, NH₄Cl, Na₂HPO₄ etc.) to provide essential elements such as magnesium, nitrogen, phosphorus and sulfur, and, optionally, amino acids.

The term "essentially consists of", as used herein, is intended to mean that substances other than those indicated are only contained in relatively low amounts of, e.g., less than 20 wt.%, 15 wt.%, 10 wt.%, 5 wt.% or less than 1 wt.%. For example, the luciferase may be used in lyophilized form comprising other ingredients added for, e.g., stabilization such as BSA (bovine serum albumin) and a saccharide such as sucrose. Thus, if, for example, 10 wt.% of lyophilized luciferase material is included in the composition and said lyophilized material contains 1 wt.% luciferase enzyme and 99 wt.% of other substances, the composition of the present invention contain 9.9 wt.% of substances other than those explicitly indicated.

An exemplary composition for preparing a liquid growth medium comprises 7.57 wt.% lyophilizate of luciferase (X-Shining^{™} Luciferase), 37 wt.% peptone, 15 wt.% yeast extract, 7.5 wt.% meat extract, 15.6 wt.% 3-(N-morpholino)propanesulfonic acid, 17.3 wt.% 3-(N-morpholino)propanesulfonic acid, sodium salt, and 0.23 wt.% D-luciferin. An exemplary composition for preparing a solid agar growth medium comprises 3.65 wt.% lyophilizate of luciferase (X-Shining luciferase), 18.2 wt.% peptone, 7.3 wt.% yeast extract, 3.65 wt.% meat extract, 7.6 wt.% 3-(N-morpholino)propanesulfonic acid, 8.5 wt.% 3-(N-morpholino) propanesulfonic acid, sodium salt, 51 wt.% agar, and 0.1 wt.% D-luciferin.

The solid composition may be provided in various forms and is not limited to a particular solid form. However, exemplary suitable solid forms include, but are not limited to, powders, beads, micronized particles, tablets, coatings, cuttable strips, pearls and the like. Particularly suitable for use herein are powders and tablets.

Since the pre-formulated composition of the present invention generally contains all ingredients required for medium preparation and for luciferase-based ATP detection it can be conveniently mixed with water for immediate use as a medium. Importantly, the luciferase-containing composition can be added to, or mixed with, water only once at the beginning of the experiment, measurement or monitoring. This is, no fresh luciferase enzyme needs to be added throughout the experiment, measurement or monitoring, and the luminescence can be measured continuously (real-time) directly from the growing cell culture.

Also, the pre-formulated composition of the present invention can be easily stored for extended periods of time prior to use without allowing for significant deterioration of its ingredients. Thus, the composition according to the first aspect of the present invention has an advantageous combination of stability during storage and ease of use.

In a second aspect, the present invention relates to the use of the solid composition according to the present invention in a method for the detection of ATP from cell cultures.

Advantageously, the method involves the measurement of luminescence continuously and directly from a growing cell culture.

The solid composition according to the first aspect of the present invention is a pre-formulated medium that, when mixed with an aqueous solution such as water, provides a medium for, e.g., the detection of ATP from cultures and, particularly, for assessing cell viability, as explained in detail below. Furthermore, the solid composition according to the first aspect of the present invention may further contain an antibiotic. In this case, the solid composition can be used for assessing the susceptibility of bacterial cells to the antibiotic, as also explained in detail below.

An important advantage of the methods described below is that the luciferase enzyme can be added directly to conventional growth media, thereby enabling online (real-time) assay formats. This means that the level of (bio)luminescence generated by the action of the luciferase enzyme, which correlates with cell growth, can be conveniently measured, either continuously or intermittently, in real-time during culturing and growth of the cells. Furthermore, no lysis of cells is required as compared to standard endpoint assays, which renders the methods of the present invention less burdensome, simpler and less expensive.

Moreover, the luciferase-based method for assessing susceptibility of bacterial cells to antibiotics (method of antibiotic susceptibility testing (AST)) is based on the surprising finding that bacteria (e.g., *E. coli*), despite a lack of cell growth, release ATP faster and in greater quantities when exposed to the antibiotic (e.g., ampicillin) than bacteria that are not exposed to the antibiotic. Bacterial strains that are resistant to the antibiotic show no such behavior.

The advantages of the AST method of the present invention lie in the simplicity and reduced test time. The short test time is a key advantage of the present invention because time is critically important in antibiotic susceptibility testing to ensure effective antibiotic treatment in a timely manner. In addition, rapid AST results allows for quicker administration of narrow-spectrum antibiotics, which are generally less expensive and have a lower risk of side effects and inducing antibiotic resistance.

More specifically, the solid composition of the present invention can be used in a method for the detection of ATP from cell cultures, wherein the method is a method for assessing cell viability, comprising the following steps:
(a) applying a sample containing cells on a solid agar-based growth medium made by mixing an aqueous solution with the solid composition of the present invention, wherein the cells are bacterial or fungal cells,
(b) incubating the cells on the solid agar-based growth medium obtained in step (a) to grow the cells, resulting in a culture reaction mixture, and
(c) measuring luminescence during growth of the cells directly from said culture reaction mixture, wherein the measured luminescence is an indicator of adenosine triphosphate (ATP) generated by the cells that is utilized by the luciferase enzyme as a co-substrate.

The method for assessing cell viability is based on the principle that the level of cellular ATP in the medium correlates with the detectable level of luminescence generated by the luciferin-luciferase system that requires ATP as a co-substrate (ATP-based cell viability method). The term "cellular ATP", as used herein, refers to cell-derived ATP that is synthesized by the cells during growth (e.g., in step (b) of the method). External ATP, i.e., ATP added to the medium or ATP present in the sample (e.g., as a contaminant), is not covered by the term "cellular ATP". It is believed that the cellular ATP in the medium is the result of leakage of intracellular ATP into the surrounding medium. Although the amount of leaked ATP is low, it is sufficient to result in a reliably detectable luminescent signal.

As used herein, the term "cell viability" or "viability" refers to the metabolic activity of living cells, which can be assessed, e.g., by measuring adenosine triphosphate (ATP). ATP is present in all living cells and its intracellular concentration is highly regulated and maintained at a similar level in all cells. When a cell dies, the ATP is completely degraded. Thus, the ATP levels not only reflect the presence of living cells, but also correlate with the number of living cells.

The term "living cells" is generally used interchangeably herein with "live cells" or "viable cells" and refers to cells that are metabolically active (e.g., produce ATP) and are generally able to grow. In connection with the present invention, the terms "cell growth" and "proliferation" may be used interchangeably used and refer to the increase in cell number.

The "cells" used herein are not particularly limited and generally include one or more of single cells, cells comprising cellular aggregates, or an organized structure or network of cells forming a tissue. In the context of the present invention, the cell(s) is/are bacterial cell(s) or fungal cell(s) including yeast and mold cells. Preferably, the cells are bacterial cells.

The bacterial cells may be selected from the group consisting of *Salmonella* spp. (e.g., *S*. *enterica*), *Listeria* spp. (e.g., *L. monocytogenes*), *Staphylococcus* spp. (e.g., *S*. *aureus*), *Streptococcus* spp. (e.g., *S*. *pyogenes, S. agalactiae*), *Pseudomonas* spp. (e.g., *P. aeruginosa*), *Klebsiella* spp. (e.g., *K. pneumonia*), *Camplylobacter* spp. (e.g., *C. jejuni, C. coli, C. lari*), *Bacillus* spp. (e.g., *B. cereus*), *Clostridium* spp. (e.g., *C. perfringens, C. difficile*), *Mycobacterium* spp. (e.g., M. *tuberculosis), Enterococcus* spp. (e.g. *E. faecalis), Citrobacter* spp. (e.g., *C*. *diversus*), *Vibrio* spp. (e.g., *V. cholerae), Prevotella* spp. (e.g., *P. brevis), Shigella* spp. (e.g., S. *flexneri), Legionella* spp. (e.g., *L. pneumophilia), Escherichia* spp. (e.g., *E*. *coli).* Preferably, the bacterial cells are *Staphylococcus* spp., *Salmonella* spp., or *Escherichia coli.*

The fungal cells may be selected from *Aspergillus* spp. *(e.g., A. niger)* and yeasts including *Candida* spp. (e.g., *C. albicans*), *Saccharomyces* spp. (e.g., *S*. *cerevisiae*) and *Pichia* spp. (e.g., *P. kluyveri*)*.* Preferably, the fungal cells are selected from *Candida* spp., *Saccharomyces* spp., and *Aspergillus* spp.

In step (a) of the method for assessing cell viability, a sample containing cells (i.e., bacterial cells or fungal cells) is applied on a solid growth medium made by mixing an aqueous solution with the solid composition according to the present invention. The fact that the luciferase enzyme and luciferase substrate are present in the solid growth medium is an important aspect since this allows for the real-time determination of ATP during culturing/growth of the cells by means of measuring the luminescence signal.

Within the present invention, the term "growth medium" is used herein to mean a liquid solution which is used to provide sufficient nutrients (e.g., carbon- and energy sources, vitamins, amino acids, essential nutrients, salts, and the like) and properties (e.g., osmolarity, buffering) to maintain live cells, such as bacterial and fungal cells, and support their growth. The growth medium may herein also referred to as "nutrient growth medium". The term "solid growth medium", as used herein, is intended to include solid as well as semi-solid growth media like a gel-like growth medium (e.g., an agar-based medium). Preferably, the solid growth medium used herein comprises agar, i.e., the solid growth medium is preferably an agar growth medium. A solid growth medium may also include film formats where the culture medium is on a foil (like Petrifilm^{™} of 3M).

The term "agar", as used herein, means a gelatinous substance, generally derived from algae. In particular, the term "agar" as used herein may refer to one or more members of a family of compounds derived from polysaccharide agarose, which forms the cell walls of algae called agarophytes. In most instances, agar includes a mixture of two polysaccharides, i.e., agarose and agaropectin, with agarose generally making up a higher proportion of the mixture. Agarose is a linear polymer, made up of repeating units of agarobiose, a disaccharide made up of D-galactose and 3,6-anhydro-L-galactopyranose. Agaropectin is a heterogeneous mixture of smaller molecules, and is made up of alternating units of D-galactose and L-galactose heavily modified with acidic side-groups, such as sulfate and pyruvate.

The solid growth medium used in the present invention may comprise peptone, meat extract, yeast extract, or any mixture of two or more thereof. Suitable growth media for use herein include, without limitations, common commercially prepared media such as Nutrient Agar, Luria Bertani (LB) agar and broth, Mac Conkey agar, Lauryl Sulfate Broth, Sabouraud Dextrose (SD) agar and broth, and Yeast medium (YM) broth.

The D-luciferin may be used in any form, e.g., in its free acid form or in its salt from. Preferably, the D-luciferin is used in its free acid form.

Within the framework of the present invention, the luciferase enzyme is generally a thermostable luciferase. The term "thermostable" as used herein in connection with the luciferase enzyme means that the luciferase is resistant to irreversible inactivation (e.g., due to irreversible changes in its chemical structure such as denaturation) at elevated temperatures, particularly at a temperature of 30-45°C or 35-40°C, and more particularly at about 37°C.

Further, the thermostable luciferase is preferably a modified firefly luciferase, in particular a modified luciferase derived from (wild-type) *Photuris pennsylvanica* or *Photinus pyralis* luciferase. In particular, the thermostable luciferase is preferably derived from the luciferase of *P. pennsylvanica* and, more preferably, has an amino acid identity of at least 80%, in particular at least 85%, at least 90% relative to the wild-type amino acid sequence of luciferase from *P. pennsylvanica.*

As used herein the term "amino acid identity" of two related amino acid sequences, expressed as a percentage, refers to the number of positions in the two optimally aligned sequences which have identical residues (x 100) divided by the number of positions compared. A gap, i.e., a position in an alignment where a residue is present in one sequence but not in the other, is regarded as a position with non-identical residues. The "optimal alignment" of two sequences is found by aligning the two sequences over the entire length. Two identical sequences have a sequence identity of 100%. Aligned sequences of amino acid residues are typically represented as rows within a matrix. Gaps are inserted between the residues so that identical or similar characters are aligned in successive columns. In order to determine the sequence identity the Needleman and Wunsch global alignment algorithm (Needleman and Wunsch, 1970, J. Mol. Biol. 48(3):443-453) of the European Molecular Biology Open Software Suite (EMBOSS, Rice et al., 2000, Trends in Genetics 16(6): 276-277; see, e.g., http://www.ebi.ac.uk/emboss/align/index.html) using default settings (gap opening penalty = 10 (for proteins) and gap extension penalty = 0.5 (for proteins)) can be employed. EBLOSUM62 can be used as the default scoring matrix.

Preferably, the thermostable luciferase used in the present invention is a luciferase enzyme that retains at least 20%, at least 40%, at least 70%, at least 80%, at least 90% or at least 95% activity after incubation for 1 hour at 60°C in an aqueous buffer solution at pH 7.8. In particular, the thermostable luciferase used herein is a luciferase enzyme characterized in that it retains at least 20%, at least 40%, at least 70%, at least 80%, at least 90% or at least 95% activity after incubation for 1 hour at 60°C in a buffer comprising 50 mM tris(hydroxymethyl)aminomethane, 10 mM MgSO₄·7H₂O, 2.0 g/l bovine serum albumin (BSA), 6 mM D/L-cystein, 1 mM ethylenediaminetetraacetic acid (EDTA), and 20 µm tetrasodium pyrophosphate, wherein the pH is adjusted to 7.8 with acetic acid.

A "cell sample" within the meaning of the present invention relates to any material, substance or composition comprising cells. The cells may be as defined above and can be derived from any source. The cell sample may be collected using a cell collection device such as a swab, broom, brush, spatula, or similar collection devices or by collecting a cell-containing material or composition (e.g., a liquid). Accordingly, basically any sample may be used in which cells may be present including, without limitations, cell suspensions, a cell containing specimen or culture (e.g., cellular and tissue cultures, pre-cultured cell cultures in growth medium), clinical and laboratory biological samples, food (e.g., liquid and solid food products, and food waste) and agricultural samples, drinking and recreational water samples.

In case of solid growth media (e.g., agar plates), the solid growth media can be inoculated directly from surfaces or even by particles from the air. Often samples are also resuspended in buffer and then plated out (e.g., food samples) or aqueous samples are plated out (drinking water) or filters are applied on which bacteria from larger liquid volumes have been concentrated. The cells may also be comprised in a liquid which can at least partially originate from the biological sample itself (e.g., in the case of a cell culture) or can be partially or fully provided by a collection medium that is used to receive the cells and/or that is used to receive the cell-containing biological sample. For example, a cell sample that was obtained by using a swab or other cell collection device (such as spatula or brush) can be contacted with a collection medium. Cells comprised in or cell samples contacted with a respective collection and/or storage medium are also encompassed by the term "cell sample" as used herein.

In accordance with the method for assessing cell viability, the "contacting" of step (a) can be carried out in any manner as long as the cells contained in the sample are brought into contact with, mixed in or added to the growth media.

In step (b), the cells on the solid growth medium are incubated to grow the cells, resulting in a culture reaction mixture. Suitable incubating conditions for growing different types of cells are well-known in the art. The incubation time depends on various factors, including the number of initial cells, the type of cells (e.g., the bacterial or fungal species), the type of growth medium and culture conditions. However, the incubation time must at least be sufficiently long so as to result in a luminescence signal that is significantly increased compared to a control sample.

Advantageously, non-microbial ATP (e.g., from food) that might be present at the beginning of step (b) is used by the luciferase enzyme before growth-related microbial ATP starts to accumulate. This means that only cellular ATP is detected, which correlates with the number of viable cells. Further, no ATP digesting enzyme needs to be added to remove unwanted external ATP, making the assay format as simple as possible.

In step (c), luminescence during growth of the cells is directly measured from the culture reaction mixture obtained in step (b). Advantageously, no lysis of cells is required prior to measuring the luminescence. This reduces the number of steps and allows for the real-time monitoring of the luminescence. The measured luminescence is an indicator of ATP released by the cells that is utilized by the luciferase enzyme as a co-substrate. Since the ATP level reflects the presence of live cells, an increase of luminescence over time indicates growth of cells, i.e., increase in the number of live cells, and thus cell activity (metabolic activity).

The luminescence can be measured using a standard luminometer (e.g., a luminescence microplate reader, a tube luminometer or an imaging device with a cooled CCD camera) as known in the art. The luminescence readings are expressed as RLU (relative luminescence units). The luminescence can be continuously measured or, alternatively, be measured from time to time, i.e., intermittently, as desired.

Moreover, the solid composition can be used in a method for assessing the susceptibility of bacterial cells to antibiotics, as mentioned above. Specifically, it can be used in a method for assessing susceptibility of bacterial cells to antibiotics, comprising the following steps:
(a) applying a sample containing bacterial cells on a solid growth medium, the solid growth medium comprising a luciferase enzyme, an antibiotic and either D-luciferin or a pro-luciferin,
(b) incubating the bacterial cells on the solid growth medium obtained in step (a) to result in an incubation mixture, and
(c) measuring luminescence during incubation of the bacterial cells directly from said incubation mixture, wherein the measured luminescence is an indicator of adenosine triphosphate (ATP) released by the cells that is utilized by the luciferase enzyme as a co-substrate.

This method may also be referred to as "antibiotic susceptibility testing" or "AST method" and is based on online (or "real-time") measurement of ATP release. It can be used for the rapid assessment of susceptibility of live bacterial cells to antibiotics. The term "susceptibility", as used herein, is intended to mean a physiological response of a microorganism (e.g., bacterial or fungal cells) to an antimicrobial agent (i.e., an antibiotic). The desired physiological response to an antibiotic is one which adversely affects the viability of the microorganism, which results in partial or complete inhibition of growth and/or partial or complete elimination (i.e., death), depending on the concentration of the antibiotic.

Advantageously, the test time required for exposing the bacteria to antibiotics and measuring ATP release in accordance with the method for assessing the susceptibility of bacterial cells to antibiotics is very short (e.g., about 0.5 to 1 hour). It does not rely on determination of time-consuming growth inhibition but measures the amounts of released ATP. In addition, the method is sufficient sensitive so that, for certain medical samples, depending on the cell concentration, enrichment of cells by incubation with medium may not be necessary. Generally, 4 to 8 hours, and up to a maximum of 12 hours in certain cases, is enough time for growing the cells to obtain a sufficiently high cell count of living cells for the AST method. The reduced test time of the method allows for quicker administration of narrow-spectrum antibiotics, which leads to improved patient outcomes, lower costs and reduced risk of antibiotic resistant.

Steps (a), (b) and (c) of the AST method are essentially identical to steps (a) and (b) of the method for assessing cell viability described above, except for the presence of an antibiotic, and the comments, explanations and definitions provided above in connection with the method for assessing cell viability equally apply here.

The antibiotic used in the AST method of the present invention is selected from β-lactam antibiotics including, but not restricted to, penicillins such as methicillin, cephalosporines such as cefotaxime, and carbapenems such as imipenem, glycopeptide antibiotics such as vancomycin, aminoglycoside antibiotics such as neomycin, protein translation inhibitors such as chloramphenicol, and polypeptide antibiotics such as colistin.

Generally, the AST method is based on ATP detection with luciferase and D-luciferin as substrate. If a pro-luciferin is used, the pro-luciferin is converted to D-luciferin or D-aminoluciferin by a specific enzyme activity of the target bacteria (target bacterial cells). Hence, the method using a pro-luciferin allows one to assess the susceptibility to antibiotics and, at the same time, to specifically detect certain bacterial species. More specifically, if there is a rapid increase in luminescence, this means that there is both enzyme activity (it is a specific type of bacterium) and release of ATP (this strain reacts to the antibiotic at the concentration used).

The AST method may further comprise step (d) of carrying out steps (a), (b) and (c) without an antibiotic (i.e., in the absence of an antibiotic) as a control. Moreover, the method may comprise steps (a) to (d) and further step (e) of comparing the luminescence measured in step (c) with the luminescence measured in step (d) without an antibiotic. Furthermore, the method may comprise a step of determining the MIC (minimal inhibitory concentration) of the antibiotic from the luminescence measured over time in step (c).

The present invention will now be further illustrated by the following, nonlimiting examples.

### EXAMPLES

The examples provided below show that a thermostable luciferase can be added directly to conventional growth media and used (a) in a real-time ATP assay for the detection of live bacterial cells and (b) in an ATP assay for antibiotic susceptibility testing (AST).

### EXAMPLE 1

### ATP assay for the detection of live bacterial cells on solid medium

Nutrient agar pH 7.4 (5 g/l peptone, 5 g/l NaCl, 2 g/l yeast extract, 1 g/l meat extract, 15 g/l agar) was autoclaved and cooled to 50°C. 1:1000 v/v of a thermostable luciferase (X-Shining^{™} Luciferase; aqueous solution with glycerol, 10 mg/ml; available from Biosynth AG, Switzerland) was added to a final concentration of 10 µg/ml. Next, 40 µM D-luciferin (Biosynth AG) was added from a concentrated stock solution. The prepared agar with luciferase enzyme and its substrate D-luciferin was then added to a white microplate (0.2 ml per well) and the plate was left for solidification for several hours.

Actively growing cultures (Nutrient Broth, 37°C, OD₆₀₀ 0.08 to 0.33) of *Staphylococcus aureus* ATCC 29213 and *Salmonella enteritidis* RKI 05/07992 were then diluted to 10⁴ CFU in sterile PBS and inoculated to microplate wells with agar (1 µl per well = approx. 10 CFU/well). 1 µl sterile phosphate buffered saline (PBS) was added to sterile control wells. The surrounding wells were filled with sterile water and the plate was incubated at 37°C in a luminescence microplate reader. The luminescence was measured every 10 min (kinetic mode).

The results are shown in **FIG. 1** and demonstrate that thermostable luciferase can be added directly to growth media, thereby enabling online (real-time) ATP-based assays for assessing cell viability (i.e., the presence of live cells). A significant luminescence increase can be measured after about 7 to 10 hours for *Salmonella enteritidis* (closed circles) and *Staphylococcus aureus* (open triangles), when starting with approximately 10 cells. Hence, cell viability can be conveniently assessed within a short period of time.

Furthermore, for hygiene tests, the presence of luciferase in the growth medium from the beginning of incubation has the advantage that non-microbial ATP (e.g., from food) is consumed by the luciferase enzyme before growth-related microbial ATP starts to accumulate. Thus, only cellular ATP is detected, which render the method more reliable. Further, no ATP digesting enzyme needs to be added to remove unwanted external ATP, making the method as simple as possible.

### EXAMPLE 2

### ATP assay for the detection of live bacterial cells in liquid medium

Nutrient broth pH 7.4 (5 g/l peptone, 5 g/l NaCl, 2 g/l yeast extract, 1 g/l meat extract) was autoclaved and cooled to room temperature. 1:1000 v/v of the same thermostable luciferase as in Example 1 was added to a final concentration of 10 µg/ml. Next, 0.4 mM D-luciferin (Biosynth AG) and 1 mM MgCl₂ was added from concentrated stock solutions. The prepared broth was sterilized by filtration (0.2 µm), incubated for 2.5 h at 30°C in order to burn-off background and then added to a white microplate (0.198 ml per well).

Agar plate pre-cultures of *Pseudomonas fluorescens* ATCC 49838 (**FIG. 2A**), *Pantoea agglomerans* RKI 16-2 (**FIG. 2B**), *Bacillus cereus* ATCC 14579 (**FIG. 2C**), *Staphylococcus aureus* ATCC 29213 (**FIG. 2D**) and *Escherichia coli* ATCC 25922 (**FIG. 2E**) were resuspended in sterile PBS, serially diluted in the same diluent and then inoculated to the microplate (2 µl per well). An optical density (OD, 600 nm) of 0.1 was considered to represent a cell concentration of 10⁸ CFU/ml for all strains. Initial cell concentrations in microplate wells were as follows: 10⁶ CFU/ml (closed circles), 10⁵ CFU/ml (open circles), 10⁴ CFU/ml (closed triangles), 10³ CFU/ml (open squares), 10² CFU/ml (closed squares), 0 (sterile control, open triangles). The microplate was covered with a transparent lid and incubated at 30°C in a plate reader, relative light units (RLU) were measured automatically every 10 min.

In a second, similar experiment, equal volumes of cell suspensions (OD 0.1) of all five stains were mixed, serially diluted in sterile PBS and then inoculated to a similar microplate (2 µl per well, 3 replicate mixtures and dilution series). Cell concentration of mixtures were determined by plating on tryptic soy agar (**FIG. 2F**). Initial cell concentrations in microplate wells were: 6·10⁵ CFU/ml (closed circles), 6·10⁴ CFU/ml (open circles), 6·10³ CFU/ml (closed triangles), 6·10² CFU/ml (open squares), 6·10¹ CFU/ml (closed squares), 0 (sterile control, open triangles).

The results of **FIGS. 2A-F** show that all tested bacterial strains, including both Gram negative and Gram positive bacteria, exhibit an exponential increase in relative light units (RLU) which then levels off or decreases. The time point when exponential increase of RLU starts (onset time) depends on initial cell concentration. The dynamics of RLU increase and dependency of onset time on initial cell concentration also apply to the mixture of bacterial species.

### EXAMPLE 3

### Correlation of luminescence onset time and cell concentration

The luminescence onset times were deduced from the time courses of the luminescence presented in FIGS. 2A-F. The "onset time" was defined as the first time point after start of incubation when the following criteria were fulfilled: (i) rate of RLU increase per hour for four consecutive time points is higher than 150, (ii) rate of RLU increase does not drop below 100 per hour for the next three time points.

The onset time was plotted against initial cell concentration (viable cells) in log scale, and the regression coefficient and formula of a fitted exponential function were calculated in Excel (**FIG. 3A**: *P. fluorescens*, **FIG. 3B**: *P. agglomerans*, **FIG. 3C**: *B. cereus*, **FIG. 3D**: *S*. *aureus*, **FIG. 3E**: *E. coli*, **FIG. 3F**: mixture of all five strains (average values of three replicate experiments using the mixture of all five strains, error bars represent standard deviations).

The results shown in **FIGS. 3A-F** demonstrate that initial cell concentration (viable cells) of all tested bacterial strains negatively correlate with onset time of luminescence increase. Accuracy of correlation of the presented method is in a range that allows quantification of the concentration of microbial cells in a sample which are capable of multiplication (viable cell count).

### EXAMPLE 4

### Relative activity of thermostable luciferase in different growth media and buffers commonly used in biological assays

The relative activity of thermostable luciferase in different growth media and buffers commonly used in biological assays was analyzed at room temperature (20-23°C). Luminescence (relative light units, RLU) was measured with a tube luminometer, thermostable luciferase (same as used in Example 1) was added at 10 µg/ml final concentration, total assay volume was 0.2 ml, enzymatic reaction was started by adding 0.15 mM D-luciferin and 0.4 µM ATP from 20x concentrated stock solution.

All buffers and media except reference and except when noted otherwise were supplemented with 1 mM MgCl₂ (some Mg²⁺ is introduced with the enzyme solution into both the supplemented and non-supplemented buffers and media). The measurement temperature was room temperature. The results are shown in Table 1 (D-Luc = D-luciferin).

**Table 1.**

| Buffer/medium | Initial luminescence | | Luminescence after 1 day |
|---|---|---|---|
| | Background D-Luc | ATP and D-Luc | ATP and D-Luc |
| Tris acetate luciferase assay buffer with bovine serum albumin and MgSO₄, pH 7.8 (Reference Buffer) | 2'340 | 7'959'877 | - |
| Nutrient Broth pH 7.4 | 24'766 | 4' 138' 964 | 5' 118'734 |
| Nutrient Broth pH 7.4 without additional MgCl₂ | 25'947 | 4'144'403 | 3' 149'287 |
| Luria Bertani broth pH 7.0 | 4'179 | 1'389'838 | 873'268 |
| Luria Bertani broth pH 7.0 without additional MgCl₂ | 1'896 | 826'554 | 501 '136 |
| Yeast extract peptone dextrose broth (YPD), pH 6.5 | 48 | 14'013 | 7'015 |
| 50 mM Tris HCl pH 7.5 | 301 | 13'685'060 | 12'035'024 |
| 50 mM Tris HCl pH 7.5 without additional MgCl₂ | 27 | 1'517'673 | 193'547 |

The results demonstrate that luciferase retains significant activity in numerous commonly used assay buffers and growth media for at least one day at 37°C. In case of defined buffer solutions, it is advantageous to add magnesium salts. In most cases the enzyme retains > 50% activity within 24 h at room temperature. Thus, as shown in Examples 1 and 2, thermostable luciferase is suitable for measuring luminescence directly from cell-containing growth media in real-time without the need for cell lysis and without using a particular ATP-assay buffer.

### EXAMPLE 5

### ATP assay for antibiotic susceptibility testing

Nutrient broth with luciferase, D-luciferin and MgCl₂ was prepared in a similar way as in Example 2 and filled in wells of a white microplate (0.19 ml per well). Ampicillin was added from a dilution series of a filter-sterilized, concentrated stock solution prepared in sterile ultrapure water (5 µl per well). Sterile water was added to control wells without antibiotic. Wells were inoculated with 5 µl of cell suspensions (OD 0.1, prepared in sterile PBS from agar plate pre-cultures) of either Ampicillin resistant (AMP^{R}) *E. coli* RKI 66/09 (AmpC) or Ampicillin sensitive (AMP^{S}) *E. coli* ATCC 25922. Initial cell concentration in wells was approximately 2.5×10⁶ CFU/ml. The microplate was incubated at 37°C in a luminescence microplate reader and relative luminescence units (RLU) were measured every 2 min for 8 h. For comparison, optical density (600 nm) was measured for all wells after 15 h incubation at 37°C.

The results of **FIGS. 4A** and **4B** show that time course of ATP release by growing cells is independent of ampicillin concentration in case of ampicillin-resistant (AMP^{R}) *E*. *coli.* In contrast, in case of AMP-susceptible (AMP^{S}) *E*. *coli*, time course and intensity of ATP release depends on the ampicillin concentration. More specifically, the results of **FIG. 4B** show that ATP release occurs earlier and is increased compared to the control (0 µg/ml ampicillin; crosses) when antibiotic concentration is at the minimal inhibitory concentration (MIC) (6.25 µg/ml ampicillin; shaded circles) or above the MIC (100 µg/ml (filled circles), 25 µg/ml (open circles). At non-inhibitory concentrations (1.56 µg/ml; filled triangles), the ATP release is slower than at or above the MIC, but very high amounts of ATP are released as indicated by the strong increase in RLU. Without being bound by theory, this may be due to the fact that at non-inhibitory concentrations, there is still some bacterial growth (as shown in FIG. 4D), but the cells release high amounts of ATP due to the antibiotic effect.

**FIG. 4C** shows the onset time (first time point with RLU above 1200) in dependency of ampicillin concentration for AMP^{R} *E*. *coli* (closed circles) and AMP^{S} *E. coli* (open circles). As can be seen, the onset time inversely correlates with ampicillin concentration for AMP^{S} *E*. *coli,* whereas essentially the same onset time is observed for all tested ampicillin concentrations in case of AMP^{R} E. *coli.* Further, ATP increase at inhibitory antibiotic concentrations can already be detected after 30 min at cell sample concentrations as low as 2×10⁶ CFU/ml. In addition, a MIC of about 6 µg/ml can be derived (see first open circle without further decrease in onset time in FIG. 4C).

**FIG. 4D** shows the optical density after 15 h at different ampicillin concentration for AMP^{R} *E*. *coli* (closed circles) and AMP^{S} *E*. *coli* (open circles). The growth of bacteria (as indicated by the optical density) at different ampicillin concentrations shows good agreement with the onset time at different ampicillin concentrations (FIG. 4C). As for the data of FIG. 4C, a MIC of about 6 µg/ml can be derived (see first open circle without further decrease in onset time in FIG. 4C; first open circle with OD at 0 in FIG. 4D). In contrast, in the resistant strain, in which there was no growth inhibition at all tested ampicillin concentrations (FIG. 4D, black circles), there was no shift to shorter onset times (= time at which 1200 RLU is reached) at increasing ampicillin concentrations and no increased ATP release.

Having regard to the above, the results show that despite the lack of cell growth in the sensitive strain (shown in Fig. 4D, white circles at 10-100 µg/ml ampicillin), ATP is released faster and in larger amounts in the presence of the antibiotic than in the control culture without antibiotic (see FIG. 4B and FIG. 4C). If the antibiotic is effective, there is a faster and stronger release of ATP than in the control without antibiotic. If the antibiotic is not effective (as in the resistant strain), there is no difference with and without antibiotic. Furthermore, based on the measured luminescence curves, the minimum inhibitory concentration (MIC) can be determined which is helpful in selecting an effective antibiotic.

The above results further show that the AST test time is reduced since low initial concentrations of bacteria can be used, thereby reducing the time needed for growing the isolated bacteria to a suitable concentration for testing. In addition, the test time required for exposing the bacteria to antibiotics and measuring ATP release is very short (e.g., 0.5 to 1 hour) and does not rely on bacterial growth. Reduced time is critically important since it allows for quicker administration of narrow-spectrum antibiotics, which leads to improved patient outcomes, lower costs and reduced antibiotic resistant bacteria strains. Thus, the presented AST method based on the measurement of ATP is suitable for rapid testing of efficacy of antibiotics.

## Claims

1. A solid composition for preparing a solid agar growth medium by mixing the solid composition with an aqueous solution, the solid composition comprising luciferase, D-luciferin, nutrients, and agar.

2. The solid composition of claim 1, wherein the nutrients include a carbon source and salts.

3. The solid composition of claim 2, wherein the nutrients include a carbon source, salts, and amino acids.

4. The solid composition of claim 1, wherein the nutrients include one or two or all three of peptone, yeast extract, and meat extract.

5. The solid composition of any one of claims 1 to 4, further comprising a buffer substance.

6. The solid composition of claim 1, comprising 0.01-0.10 wt.% luciferase, 0.02-0.40 wt.% D-luciferin, 30-70 wt.% agar, at least one of peptone, yeast extract and meat extract, and, optionally, a buffer substance

7. The solid composition of any one of claims 1 to 6, wherein the solid composition consists of less than 20 wt.% of substances other than those mentioned in any one of claims 1 to 6.

8. The solid composition of any one of claims 1 to 7, wherein the solid composition is in the form of a powder, bead, micronized particle, tablet, coating, cuttable strip, or pearl.

9. Use of the solid composition of any one of claims 1 to 8 in a method for the detection of ATP from cell cultures.

10. The use of claim 9, wherein the method involves the measurement of luminescence continuously and directly from a growing cell culture.

11. The use of claim 9 or 10, wherein the method is a method for assessing cell viability, comprising the following steps:
(a) applying a sample containing cells on a solid agar-based growth medium made by mixing an aqueous solution with the solid composition according to any one of claims 1 to 8,
wherein the cells are bacterial or fungal cells,
(b) incubating the cells on the solid agar-based growth medium obtained in step (a) to grow the cells, resulting in a culture reaction mixture, and
(c) measuring luminescence during growth of the cells directly from said culture reaction mixture, wherein the measured luminescence is an indicator of adenosine triphosphate (ATP) released by the cells that is utilized by the luciferase enzyme as a co-substrate.

## Patentansprüche

1. Feste Zusammensetzung zum Herstellen eines festen Agar-Wachstumsmediums durch Mischen der festen Zusammensetzung mit einer wässrigen Lösung, wobei die feste Zusammensetzung Luciferase, D-Luciferin, Nährstoffe und Agar umfasst.

2. Feste Zusammensetzung nach Anspruch 1, wobei die Nährstoffe eine Kohlenstoffquelle und Salze enthalten.

3. Feste Zusammensetzung nach Anspruch 2, wobei die Nährstoffe eine Kohlenstoffquelle, Salze und Aminosäuren enthalten.

4. Feste Zusammensetzung nach Anspruch 1, wobei die Nährstoffe eines oder zwei oder alle drei von Pepton, Hefeextrakt und Fleischextrakt enthalten.

5. Feste Zusammensetzung nach einem der Ansprüche 1 bis 4 ferner umfassend eine Puffersubstanz.

6. Feste Zusammensetzung nach Anspruch 1, umfassend 0,01-0,10 Gew.-% Luziferase, 0,02-0,40 Gew.-% D-Luciferin, 30-70 Gew.-% Agar, mindestens eines von Pepton, Hefeextrakt und Fleischextrakt, und optional eine Puffersubstanz.

7. Feste Zusammensetzung nach einem der Ansprüche 1 bis 6, wobei die feste Zusammensetzung zu weniger als 20 Gew.-% aus anderen als den in einem der Ansprüche 1 bis 6 genannten Substanzen besteht.

8. Feste Zusammensetzung nach einem der Ansprüche 1 bis 7, wobei die feste Zusammensetzung in der Form eines Pulvers, eines Kügelchens, eines mikronisierten Partikels, einer Tablette, eines Überzugs, eines schneidbaren Streifens oder einer Perle vorliegt.

9. Verwendung der festen Zusammensetzung nach einem der Ansprüche 1 bis 8 in einem Verfahren zum Nachweis von ATP aus Zellkulturen.

10. Verwendung nach Anspruch 9, wobei das Verfahren die kontinuierliche und direkte Messung von Lumineszenz aus einer wachsenden Zellkultur beinhaltet.

11. Verwendung nach Anspruch 9 oder 10, wobei das Verfahren ein Verfahren zum Bestimmen der Zellviabilität ist, das die folgenden Schritte umfasst:
(a) Aufbringen einer zellhaltigen Probe auf ein festes Agar-basiertes Wachstumsmedium, das durch Mischen einer wässrigen Lösung mit der festen Zusammensetzung nach einem der Ansprüche 1 bis 8 hergestellt wird,
wobei die Zellen bakterielle oder pilzliche Zellen sind,
(b) Inkubieren der Zellen auf dem in Schritt (a) erhaltenen festen Agar-basierten Wachstumsmedium, um die Zellen zu züchten, was in einer Kulturreaktionsmischung resultiert, und
(c) Messen der Lumineszenz während des Wachstums der Zellen direkt aus der Kulturreaktionsmischung, wobei die gemessene Lumineszenz ein Indikator für das von den Zellen freigesetzte Adenosintriphosphat (ATP) ist, das von dem Luciferase-Enzym als Co-Substrat genutzt wird.

## Revendications

1. Composition solide pour la préparation d'un milieu de croissance solide gélosé par mélange de la composition solide avec une solution aqueuse, la composition solide comprenant de la luciférase, de la D-luciférine, des nutriments et de la gélose.

2. Composition solide selon la revendication 1, dans laquelle les nutriments comprennent une source de carbone et des sels.

3. Composition solide selon la revendication 2, dans laquelle les nutriments comprennent une source de carbone, des sels et des acides aminés.

4. Composition solide selon la revendication 1, dans laquelle les nutriments comprennent un ou deux ou la totalité des trois éléments parmi une peptone, un extrait de levure et un extrait de viande.

5. Composition solide selon l'une quelconque des revendications 1 à 4, comprenant en outre une substance tampon.

6. Composition solide selon la revendication 1, comprenant 0,01 à 0,10 % en poids de luciférase, 0,02 à 40 % en poids de D-luciférine, 30 à 70 % en poids de gélose, au moins un élément parmi une peptone, un extrait de levure et un extrait de viande, et éventuellement, une substance tampon.

7. Composition solide selon l'une quelconque des revendications 1 à 6, dans laquelle la composition solide est constituée de moins de 20 % en poids de substances autres que celles mentionnées dans l'une quelconque des revendications 1 à 6.

8. Composition solide selon l'une quelconque des revendications 1 à 7, dans laquelle la composition solide est sous la forme d'une poudre, d'une bille, d'une particule micronisée, d'un comprimé, d'un enrobage, d'une bande apte à être découpée ou d'une perle.

9. Utilisation de la composition solide selon l'une quelconque des revendications 1 à 8 dans un procédé de détection d'ATP à partir de cultures cellulaires.

10. Utilisation selon la revendication 9, dans laquelle le procédé implique la mesure de luminescence en continu et directement à partir d'une culture cellulaire en croissance.

11. Utilisation selon la revendication 9 ou 10, dans laquelle le procédé est un procédé d'évaluation de la viabilité cellulaire, comprenant les étapes suivantes :
(a) l'application d'un échantillon contenant des cellules sur un milieu de croissance solide à base de gélose préparé en mélangeant une solution aqueuse avec la composition solide selon l'une quelconque des revendications 1 à 8,
dans laquelle les cellules sont des cellules bactériennes ou fongiques,
(b) l'incubation des cellules sur le milieu de croissance solide à base de gélose obtenu à l'étape (a) pour faire croître les cellules, ce qui permet d'obtenir un mélange réactionnel de culture, et
(c) la mesure de la luminescence pendant la croissance des cellules directement à partir dudit mélange réactionnel de culture, dans laquelle la luminescence mesurée est un indicateur de l'adénosine triphosphate (ATP) libéré par les cellules et utilisé par l'enzyme luciférase en tant que co-substrat.
